# EUROPEAN PATENT SPECIFICATION

(11) **EP 2 132 339 B1**
(45) Date of publication and mention of the grant of the patent: **09.03.2011**
(21) Application number: 07727792.9
(22) Date of filing: 04.04.2007
(51) Int. Cl.: C12Q 1/68

(54) **METHOD FOR THE DETECTION OF AN ANALYTE IN BIOLOGICAL MATRIX**
VERFAHREN ZUM NACHWEIS EINES ANALYTEN IN EINER BIOLOGISCHEN MATRIX
PROCÉDÉ DE DÉTECTION D'UN ANALYTE DANS UNE MATRICE BIOLOGIQUE

(43) Date of publication of application: 16.12.2009
(73) Proprietor: Chimera Biotec GmbH, 44227 Dortmund (DE)
(72) Inventor: ADLER, Michael, 27607 Langen-Debstedt (DE)
(74) Representative: Viering, Jentschura & Partner
(86) International application number: PCT/EP2007/053323
(87) International publication number: WO 2008/122310

(56) References cited:
- US-A1- 2005 079 520
- MCKIE A ET AL: "A quantitative immuno-PCR assay for the detection of mumps-specific IgG" JOURNAL OF IMMUNOLOGICAL METHODS, ELSEVIER, AMSTERDAM, NL, vol. 270, no. 1, 1 December 2002 (2002-12-01), pages 135-141, XP004387991 ISSN: 0022-1759
- WACKER ET AL: "Magneto Immuno-PCR: A novel immunoassay based on biogenic magnetosome nanoparticles" BIOCHEMICAL AND BIOPHYSICAL RESEARCH COMMUNICATIONS, ACADEMIC PRESS INC. ORLANDO, FL, US, vol. 357, no. 2, 3 April 2007 (2007-04-03), pages 391-396, XP022055819 ISSN: 0006-291X
- NIEMEYER CHRISTOF M ET AL: "Detecting antigens by quantitative immuno-PCR." NATURE PROTOCOLS 2007, vol. 2, no. 8, 2007, pages 1918-1930, XP002450432 ISSN: 1750-2799
- ADLER M ET AL: "Detection of Rotavirus from stool samples using a standardized immuno-PCR (''Imperacer'') method with end-point and real-time detection" BIOCHEMICAL AND BIOPHYSICAL RESEARCH COMMUNICATIONS, ACADEMIC PRESS INC. ORLANDO, FL, US, vol. 333, no. 4, 12 August 2005 (2005-08-12), pages 1289-1294, XP004962314 ISSN: 0006-291X

## Description

### Field of the Invention

The present invention lies in the field of immunology, molecular biology and molecular diagnostics and relates to the Immuno-PCR (polymerase chain reaction) technique. More specifically, the present invention relates to sample preparation methods that allow background reduction and result in highly sensitive Inununo-PCR assays.

### Background of the Invention

Immunoassays where one or more antibodies are used to detect a test substance (target, analyte) in a test sample are widely known. A standard application of this technique is the Enzyme Linked Immunosorbent Assay ("ELISA"). The ELISA either uses a capture antibody immobilized on a solid surface for specifically capturing a target antigen from a complex biological matrix (sandwich immunoassay format) or the target antigen to be detected is non-specifically adsorbed to a solid surface, wherein the solid surface is typically the inside of a microtiter plate well. Unbound matrix is removed by a washing step and subsequent coupling of an enzyme-labelled detection antibody (direct ELISA) or coupling of a detection antibody specific for the target antigen followed by coupling of a secondary enzyme-labelled antibody that binds the primary antibody (indirect ELISA). The enzyme activity associated with the solid surface which is subsequently determined is directly proportional to the amount of bound antigen present and can be measured, for example, by using a chromogenic substrate for the enzyme.

The evolution of immunoassay methods increased the sensitivity of these tests by altering the detection principle. In the course of this development, the enzyme-coupled detection antibody was replaced by oligonucleotide (e.g. DNA) labelled antibodies. In these antibody-nucleic acid conjugates the oligonucleotide served as a marker that could be subsequently amplified and detected. In an application of the PCR ("polymerase chain reaction") as an exponential amplification system for nucleic acids to these antibody-based detection system (Sano et al. (2000), "Immuno-PCR: very sensitive antigen detection by means of specific antibody-DNA conjugates" Science 258(5079):120-122), the Immuno-PCR (IPCR) method was developed. The efficacy of this method was first demonstrated for the detection of Bovine Serum Albumin (BSA) as an antigen passively absorbed to an immuno-assay plate. Using an antibody specific for BSA coupled to a biotin-labeled reporter DNA plasmid by means of a protein A-avidin fusion protein, and subsequently utilizing 30 cycles of polymerase chain reaction (PCR) amplification to amplify the reporter DNA sequence, the detection of the amplicons by staining with ethidium bromide following gel electrophoresis was possible. Sano et al. reported an enhanced detection sensitivity of approximately five orders of magnitude when compared to ELISA detection. Theoretically, a nucleic acid label may be detected with extraordinary sensitivity (potentially down to a single copy) when amplified by PCR or any other available exponential nucleic acid amplification technique. However, the sequential addition of each assay component requires extensive washing to sufficiently reduce non-specifically bound material.

Moreover, this method could not be applied to biological samples due to the absence of a specific analyte capture molecule. Because of the liability of the protein A fusion protein to bind all present antibodies, including a capture antibody, this kind of IPCR reagents, consisting of a target-specific antibody coupled via the protein A-streptavidin fusion protein to a biotinylated DNA reporter sequence, did not allow performing a sandwich assay with a capture antibody. Additionally, the direct binding of the analyte to the microplate surface limits the usefulness of this kind of assay to analytes which are present in a pure solution, as other components of a complex sample, such as a typical biological matrix, will also be attached to the surface during analyte immobilization and will thus interfere with the binding of the analyte.

Accordingly, it was highly desirable to develop a sandwich immuno-PCR. A typical sandwich immunoassay utilizes two antibodies, both of which recognize and bind the target antigen. The "capture" antibody is used to coat the surface of a solid support, such as a microtiter well, bead or particle. The "reporter" antibody is labeled with a detectable label, in case of an Immuno-PCR a nucleic acid reporter molecule. Typically, the antigen is first contacted with either the reporter antibody in solution or with the capture antibody on the solid support, followed by addition of the remaining antibody. The resulting specific immune complex is bound to the surface of the solid support and consists of the antigen combined with two antibodies. Excess antigen and antibodies are removed by repeatedly washing the support. In order to minimize background, it is crucial that unbound reporter antibody is removed, whereas it is at the same time desirable to maintain the integrity of the formed immobilized complex to maximize the signal strength. Following the washing steps, the bound reporter antibody is measured via the detection molecule as an indication of the amount of antigen present.

A sandwich Immuno-PCR thus circumvents the problem of unspecific attachment of other sample components to the solid surface during the attachment step necessary for immobilizing the antigen without a capture molecule, thus allowing the analysis of complex biological samples. However, because of the liability of the protein A-avidin fusion protein to bind any antibody present, i.e. also the capture antibody, other means of attaching the DNA to the reporter antibody had to be found.

This object has been addressed by other groups, which have improved the IPCR method by substituting the protein A-streptavidin fusion protein with a biotinylated detection antibody coupled to biotinylated DNA via sequential incubation of the antibody and the DNA with streptavidin as a tetravalent biotin-binding linker molecule (Zhou et al. (1993). "Universal Immuno-PCR for ultra-sensitive target protein detection." Nucleic Acids Res 21(25): 6038-9) or direct conjugates synthesized by covalently coupling antibodies and DNA (Hendrickson et al. (1995). "High Sensitivity Multianalyte Immunoassay Using Covalent DNA-Labeled Antibodies and Polymerase Chain Reaction." Nucleic Acids Res. 23(3): 522-529). With these strategies, a sandwich IPCR using antigen-specific capture and detection antibodies which was similar to conventional ELISA analysis and therefore able to detect antigens in complex biological matrices became accessible (Maia et al. (1995). "Development of a two-site immuno-PCR assay for hepatitis B surface antigen." J. Virol. Methods 52(3): 273-86).

Immuno-PCR is nowadays used in combination with several matrices for the detection of a number of different antigens, including virus particles, tumor markers or cytokines in various body fluids (Niemeyer et al. (2005) "Immuno-PCR: high sensitivity detection of proteins by nucleic acid amplification." Trends Biotechnol. 23(4): 208-16).

Due to the enormous exponential signal amplification, however, Immuno-PCR, as used in state of the art applications, is very susceptible to background effects by unspecific binding of sample contents and the involved reagents, especially during the antigen binding step. Since theoretically single molecules of nucleic acid can be detected by PCR, failure to remove all of the non-specifically bound template DNA results in significant background compared to conventional ELISA techniques and interferes with the ability to detect minute quantities of analyte, because the signals from these unspecific binding events are amplified in IPCR, too. As well known among scientists working in this field, a background signal is, in contrast to other standard PCR techniques where signals for the negative control are typically not detectable, measured in all typical IPCR assays.

Stringent, numerous or prolonged washes to reduce non-specific binding are no solution to this problem, because they lead to a reduced signal due to elution or dissociation of antigen/antibody complexes.

The art of improving IPCR assays is thereby mainly an attempt to either decrease the unavoidable background signal or to increase specific signals induced by the analyte in order to improve the signal-to-background ratio.

The necessity of additional purification steps of the biological sample in order to decrease the background signal in the subsequent IPCR is inconvenient and labour-intensive and negates one of the main advantages of the sandwich assay approach, namely the possibility to use crude, non-purified samples. This is a major difference to e.g. PCR where at least minimal purification of the target DNA is mandatory. Accordingly, in immunoassay applications, a simpler technique for the minimization of background effects would be highly advantageous.

To date, a variety of other methods have been suggested to reduce background in both standard immunoassays and Immuno-PCR methods. Ishikawa et al. (U.S. Patent No. 5,888, 834) have proposed the technique of immune complex transfer immunoassay, in which a sandwich immune complex is released from the capturing support and re-captured onto a fresh surface. Non-specifically bound reporter components are left behind on the initial solid support. Nucleic acid hybridization assay sensitivity has also been improved by chemically eluting hybridized DNA from a solid support, leaving behind non-specifically bound reporter DNA probe (See Morrissey, et al. (1989), Anal Biochem.181 : 345-359). Complexes have also been released by the use of homopolymers, such as 200-400 poly (rU), in combination with washing in tetraalkylammonium salts (Collins et al., US Patent No. 5,702, 896).

Additionally, the composition and properties of the biological sample also directly influence the robustness of the assay performance. This is due to the fact that many biological samples are inhomogeneous and/or viscous. Additionally, the preparation of biological samples commonly requires the use of chemicals, such as various detergents and salts, or enzymes, such as, for example, nucleases or proteases. These components present in the sample can further disturb the performance of the assay.

The use of dilution buffers for the dilution of the sample prior to the assay in order to improve assay performance in biological matrices is a well-established strategy for ELISA methods and also very Promising for Immuno-PCR applications (See, for example, international patent publication WO 2005/083433). This IPCR dilution approach was successfully applied in combination with different biological matrices such as serum (Adler et al. (2005) "Adaptation and performance of an immuno-PCR assay for the quantification of Aviscumine in patient plasma samples." J Pharm Biomed Anal 39(5): 972-82) or stool (Adler et al. (2005). "Detection of Rotavirus from stool samples using a standardized immuno-PCR ("Imperacer") method with end-point and real-time detection." Biochem. Biophys. Res. Commun. 333(4): 1289-1294).

The composition of these sample dilution buffers is known to those skilled in the art for including all or some of the following compounds (Rauch et al. 2007):
a.) a buffer to control pH
b.) a detergent to minimize unspecific binding
c.) standard proteins such as BSA or milk powder as blocking compounds for unspecific binding
d.) antibodies and/or unspecific sera binding endogenous anti-animal antibodies and/or heterophilic antibodies
e.) low molecular chemical compounds for further modulating binding effects

Although Immuno-PCR has extraordinary theoretical sensitivity, the commercial success of the technique has been hampered by the problem of non-specifically bound template DNA label, which obscures the true analyte signal. This leads to the generation of false positive results and irreproducibility when attempting to detect analytes present at low levels. Some investigators have concluded that Immuno-PCR is no more sensitive than ELISA in detecting IgG due to non-specific binding (McKie,et al. (2002), J. Immunol. Meth. 261 : 167-175). It has to be kept in mind, that the remarkable sensitivity achieved by Sano et al. was demonstrated with a pure system using a cumbersome, multi-step format, which is, as already mentioned above, impractical for medical applications.

Hence, there remains a need in the field of diagnostics and biosciences for a robust assay to detect analytes present at vanishingly low levels or concentrations. Although Immuno-PCR is theoretically capable of fulfilling the long-felt need, the minimization of unspecific binding, being an imperative for highly sensitive IPCR assays, remains a problem. Therefore, there exists still a need in the art for strategies to further improve IPCR assay performance besides the known sample dilution technique.

Thus, one object of the inventors of the present invention was to provide a method for lowering the background in an Immuno-PCR while retaining the high sensitivity.

### Summary of the Invention

The present invention is directed to a method for the determination of the presence and/or amount of an analyte in a sample by an Immuno-PCR assay, wherein the method provides an improved IPCR assay performance, the method comprising the step of diluting the sample with a buffer containing nucleic acid molecules, wherein the nucleic acid molecules have the effect that unspecific binding is reduced and thus the signal-to-background ratio increased.

This improvement of the performance of an Immuno-PCR assay is based on the surprising finding of the inventors of the instant invention that the addition of nucleic acid molecules alone or in combination with protein and/or peptide molecules to the sample prior or during the incubation with a analyte-specific binding molecule can, to a certain extent, reduce unspecific binding and thus improve the performance of an Immuno-PCR assay. Thus, in a first aspect, the present invention relates to a method for determining the presence and/or amount of an analyte in a sample by an Immuno-PCR, the method including the dilution of the sample with a buffer solution, the buffer solution being characterized by that it comprises one or more nucleic acid molecules, wherein the concentration of the nucleic acid molecules in the sample dilution buffer is between about 0.01 mg/ml and about 10 mg/ml.

The dilution of the sample with the nucleic acid molecules containing buffer solution may, for example, be carried out prior to incubation of the sample with a binding molecule, e.g. an antibody, or during the contacting of the sample with a specific target analyte-binding molecule. This target analyte-binding molecule can be, for example, a capture molecule used for the immobilization of the target to a solid surface, or a detection molecule that allows the detection of the presence and/or amount of the target in the assayed sample. Both, the capture molecule and the detection molecule, are commonly target analyte-specific antibodies, with the detection antibody usually coupled to a reporter nucleic acid sequence that allows specific detection.

Accordingly, in one specific aspect, the invented method for determining the presence and/or amount of an analyte in a sample by Immuno-PCR comprises contacting the sample with a first binding molecule specifically binding the analyte, wherein the first binding molecule may optionally be immobilized on a solid surface, in the presence of a sample dilution buffer composition comprising one or more nucleic acid molecules. The first binding molecule would thus act as a capture molecule.

The thus formed complex between analyte and capture molecule can, if the first binding molecule is not already immobilized on a solid surface, then be immobilized on a solid support, such as a microtiter plate well or reaction vessel, via the capture molecule.

In another aspect of the invention, the method for determining the presence and/or amount of an analyte in a sample by Immuno-PCR comprises contacting the sample with a first binding molecule specifically binding the analyte, wherein the first binding molecule is labelled with a detectable label, in the presence of a sample dilution buffer composition comprising one or more nucleic acid molecules. The detectable label can be a nucleic acid sequence, for example a DNA sequence. The first binding molecule would thus represent the detection molecule.

In both alternatives, the sample dilution buffer containing one or more nucleic acid molecules may have been added to either the sample or the first binding molecule prior to the contacting of sample and first binding molecule. Alternatively, all three components, i.e. sample, first binding molecule and sample dilution buffer, are contacted simultaneously.

If the first binding molecule is the capture molecule, the formed complex between analyte and capture molecule is in the following contacted with the second binding molecule, i.e. the detection molecule. Between both contacting steps one or more washing steps may be carried out to remove unbound sample components. Additionally, if the complex formation occurred in solution, the immobilization of the complex on a solid support may be carried out before the incubation with the detection molecule.

In case the first binding molecule is the detection molecule, the formed complex between analyte and detection molecule is subsequently contacted with the capture molecule, which may be immobilized on a solid support. Again, one or more washing steps may be performed prior to formation of the ternary complex of capture molecule, analyte and detection molecule.

The above-described methods relate to an Immuno-PCR assay in the sandwich immunoassay format, meaning the analyte is bound by a first binding molecule which is or will be immobilized on a solid support and a second analyte-binding molecule which allows detection of the formed ternary complex.

In another embodiment, the sample is contacted with a solid support in the presence of a sample dilution buffer containing one or more nucleic acids, wherein the solid support is capable of binding the analyte. After performing optional washing steps, the immobilized analyte can then be contacted with a detection molecule. Alternatively, the detection molecule may be already present during the contacting of the sample with the solid support, either by a previous contact step between sample and detection molecule in the presence of a nucleic acid-containing sample dilution buffer or by simultaneous combination of all three components.

After the immobilization of the analyte, the capture molecule-analyte complex, the capture molecule, the detection molecule-analyte complex or the ternary complex of capture molecule, analyte and detection molecule, usually one or more washing steps are carried out to remove unbound sample components, capture molecules and/or detection molecules.

Wash buffers that are suitable for this purpose are known in the art. In a certain embodiment of the invented methods, these washing buffers also contain one or more nucleic acid molecules.

In any of the described alternatives, the solid support may be blocked with a blocking buffer, wherein this blocking buffer contains proteins/peptides and/or nucleic acid molecules. In one embodiment, the blocking can be carried out before the solid support is contacted with the analyte-containing sample. One or more washing steps may be performed to remove unbound blocking compounds prior to the contacting of the support with the sample.

In the above-described methods, the detection molecule, usually a nucleic acid-antibody conjugate, may be stored, prepared or diluted in a buffer solution that also comprises one or more nucleic acid molecules.

In addition to the afore-mentioned advantageous effect of the nucleic acid molecules contained in the sample dilution buffer on the Immuno-PCR assay performance and background reduction, the inventors have surprisingly found that the effect of the nucleic acid molecules can be synergistically enhanced if in addition one or more peptides/proteins are present in the sample dilution buffer. Hence, in the above-described methods the use of a sample dilution buffer containing one or more nucleic acid molecules and additionally one or more peptide/protein molecules is also contemplated.

The nucleic acid molecules utilized for sample stabilization, i.e. the background reduction, can be any polynucleotides. The polynucleotides may contain deoxyribonucleotides, ribonucleotides and/or their analogs and thus include, for example, DNA, RNA and peptide nucleic acid (PNA). The nucleic acid molecules may be single-, double- and/or triple-stranded. Also encompassed are hybrids of the afore-mentioned nucleic acids, such as, for example, DNA:RNA hybrids, or nucleic acid molecules comprising nucleotide derivatives. In a specific embodiment, the one or more nucleic acid molecules used in the invented methods are DNA molecules, for example fragmented genomic DNA molecules, such as fragmented fish sperm DNA. Fragmentation of the DNA may be achieved by cleavage with restriction endonucleases. If the nucleic acid molecules are added to the sample in form of a sample dilution buffer, the concentration of the nucleic acid molecules in the buffer is between about 0.01 mg/ml and about 10 mg/ml, or between about 0.1 mg/ml and about 1 mg/ml. In case the nucleic acid molecules are directly added to the sample, the final concentration of the nucleic acid molecules in the sample may range between 0.001 and 100 mg/ml, between 0.01 and 10 mg/ml, or between 0.1 and 1 mg/ml.

If in addition to the nucleic acids, the sample dilution buffer contains one or more peptides/proteins, these peptides/proteins can be of any type and include, for example, albumines, globulines and caseins. The peptides and/or proteins may be added in form of powdered milk and may be used in amounts ranging from 0.01 % to 20 % by weight, between 0.1% and 10% by weight, or between 1% and 5% by weight relative to the weight of the buffer composition.

In addition to the mentioned components nucleic acids and peptides/proteins, the sample dilution buffer can contain further compounds, such as salts and buffering substances, detergents, and chelating agents. The sample dilution buffer can be an aqueous buffer system.

In certain embodiments of the invention, washing buffers, storage buffers and dilution buffers for the binding molecules that are used in connection with the invented methods can resemble the above-described sample dilution buffer compositions or can even be identical to those.

The sample dilution with the above-described sample dilution buffers may range from about 0.1-fold to about 100-fold, from about 0.5-fold to about 10-fold, or from about 1-fold to about 5-fold.

In still another aspect, the invention also encompasses the use of a buffer solution for the dilution of a sample for the Immuno-PCR, wherein the buffer solution comprises one or more nucleic acid molecules and, optionally, one or more additional compounds selected from the group consisting of peptides, proteins, detergents, salts, buffer substances and chelating agents. This use of a buffer solution containing one or more nucleic acid molecules for dilution of an Immuno-PCR sample serves to reduce unspecific binding and thus the background of the assay.

In one embodiment, the sample to be used in the above-described methods and uses is a biological sample, including biological matrices, and can be selected from the group consisting of bodily fluids, culture media, tissue samples, and cell lysates and can originate from humans, animals, plants or microorganisms.

Also disclosed is a kit for performing Immuno-PCR assays, comprising a sample dilution buffer composition containing one or more nucleic acid molecules and optionally, one or more compounds selected from the group consisting of peptides, proteins, salts, buffer substances, detergents and chelating agents. Optionally, the kit may comprise one or more further reagents and materials selected from the group consisting of a solid support, binding molecules (in case of sandwich assays either bound to the solid support or modified for binding against a solid support which is coated with an appropriate coupling surface), detection molecule(s), wash buffers, reagents for signal amplification, a calibration solution of the target compound, positive and negative controls and instructions for use of the kit. In the kit the sample dilution buffer composition may be solid for dissolving in water prior to use or may be provided as an aqueous solution. The detection molecule can comprise an analyte-binding molecule, a reporter nucleic acid sequence, and, optionally, a linker molecule either separately or already in form of a ready-to-use Immuno-PCR conjugate. The reagents for signal amplification may include amplification primers, PCR reagents, and a dye, such as ethidium bromide or SYBR Green, for detecting the amplification product.

The nucleic acid-containing sample dilution buffer can be part of a complete kit of IPCR reagents, therefore enabling the user to perform the optimized assay with all complementary reagents and materials. An assay kit will usually contain the solid support, binding molecules (in case of sandwich assays either bound to the solid support or modified for binding against a solid support which is coated with an appropriate coupling surface), detection molecule(s), wash buffers, sample dilution buffer and a reagent solution for signal amplification. Additionally included are typically a calibration solution of the target compound as well as positive and negative controls and instructions for use of the kit. The kit may also contain further assay materials such as further vessels and foil or caps for sealing the vessel.

The kit may also be part of a complete complementary optimized system, additional including the required instruments such as washing and real-time PCR devices.

### Brief description of the drawings

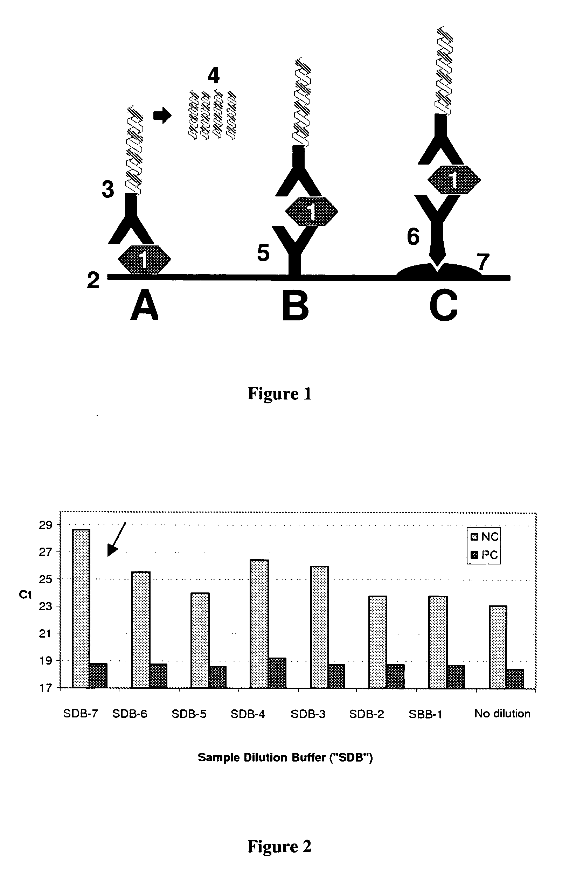

Figure 1 is a schematic drawing of a direct Immuno-PCR (A), a sandwich Immuno-PCR (B) and a sandwich Immuno-PCR using an additional coupling molecule (C). The numbers code for the following: 1: target compound, 2: solid support, 3: detection molecule, including marker nucleic acid, 4: amplified marker, 5: first ("capture") binding molecule, 6: modified first ("capture") binding molecule, 7: coupling molecule.

Figure 2 shows the results of a measurement of Ct_{PC} and Ct_{NC} values by real-time Immuno-PCR for the detection of rabbit-IgG spiked in human serum. Due to the inversed proportional signal generation, absolute signals for negative controls ("Ct_{NC}", later signal in PCR-amplification, grey) are higher than their corresponding positive control signals ("Ct_{PC}", black).

### Detailed Description of the Invention

### Definitions

The terms used herein have, unless stated otherwise, the meaning as accepted in art.

The terms "analyte", "target compound", "target molecule" or "target" as interchangeably used herein, refer to any substance that can be detected in an assay by binding to a binding molecule, and which may be present in a sample. Therefore, the analyte can be, without limitation, any substance for which there exists a naturally occurring antibody or for which an antibody can be prepared. The analyte may, for example, be a protein, a polypeptide, a hapten, a carbohydrate, a lipid, a cell or any other of a wide variety of biological or non-biological molecules, complexes or combinations thereof. Generally, the analyte will be a protein, peptide, carbohydrate or lipid derived from a biological source such as bacterial, fungal, viral, plant or animal samples. Additionally, however, the target may also be a smaller organic compound such as a drug, drug-metabolite, dye or other small molecule present in the sample.

When small molecules are the target compound, a competitive assay, including a competitive incubation step of the target compound to be analysed as present in the sample, an added modified variant of the target compound as a "competitor", and a binding molecule, can be used. In a specific embodiment of the competitive assay, the first binding molecule of the assay is specific for the target compound while the detection component is specific for the modification of the added competitor. In another embodiment, the competitor is immobilized on the solid phase and incubated with the target-containing sample and the binding molecule.

Analytes of the invention may comprise a nucleic acid component, but the binding of the analyte to be detected is not dependent on complementary hybridization between a target nucleic acid sequence in the analyte and a detection nucleic acid sequence.

The term "sample", as used herein, refers to an aliquot of material, frequently biological matrices, an aqueous solution or an aqueous suspension derived from biological material. Samples to be assayed for the presence of an analyte by the methods of the present invention include, for example, cells, tissues, homogenates, lysates, extracts, and purified or partially purified proteins and other biological molecules and mixtures thereof.

Non-limiting examples of samples typically used in the methods of the invention include human and animal body fluids such as whole blood, serum, plasma, cerebrospinal fluid, sputum, bronchial washing, bronchial aspirates, urine, semen, lymph fluids and various external secretions of the respiratory, intestinal and genitourinary tracts, tears, saliva, milk, white blood cells, myelomas and the like; biological fluids such as cell culture supernatants; tissue specimens which may or may not be fixed; and cell specimens which may or may not be fixed. The samples used in the methods of the present invention will vary based on the assay format and the nature of the tissues, cells, extracts or other materials, especially biological materials, to be assayed. Methods for preparing protein extracts from cells or samples are well known in the art and can be readily adapted in order to obtain a sample that is compatible with the methods of the invention.

A "sample matrix" as the sample material from which the target is to be detected is thereby typically - but not limited to the following - a biological sample material such as: bodily fluids (blood, plasma, serum, cerebrospinal fluid, lacrimal secretion, urine, semen, and saliva), culture media, tissue samples, and cell lysates from humans, animals, plants or microorganisms. This matrix may additionally include further interfering agents from sample preparations such as high amounts of detergents, lysis buffers and enzymes, conservation compounds etc. or insoluble compounds such as dirt or aggregated proteins.

The terms "binding molecule", "first binding molecule" and "detection molecule" as used herein refer to any molecule or target-binding fragment thereof capable of specifically binding to the target molecule so as to form a specific complex consisting of the molecule and the target. In case of the presence of a first binding molecule as described above, the detection molecule is a second binding molecule used for the specific detection of the analyte. In this case, two binding molecules are used for the specific binding of the analyte in a "sandwich" assay. During sandwich assay, the first binding molecule is also termed "capture" molecule. In case of the direct immobilization of the target against a surface without a first capture molecule, the detection molecule is the only binding molecule used for the specific binding of the analyte.

"Specifically binding" and "specific binding" as used herein mean that the binding molecule binds to the target molecule based on recognition of a binding region or epitope on the target molecule. The binding molecule preferably recognizes and binds to the target molecule with a higher binding affinity than it binds to other compounds in the sample. In various embodiments of the invention, "specifically binding" may mean that an antibody or other biological molecule, binds to a target molecule with at least about a 10⁶-fold greater affinity, preferably at least about a 10⁷-fold greater affinity, more preferably at least about a 10⁸-fold greater affinity, and most preferably at least about a 10⁹-fold greater affinity than it binds molecules unrelated to the target molecule. Typically, specific binding refers to affinities in the range of about 10⁶-fold to about 10⁹-fold greater than non-specific binding. In some embodiments, specific binding may be characterized by affinities greater than 10⁹-fold over non-specific binding. In a specific embodiment, the binding molecule uniquely recognizes and binds to the target molecule.

Typically, the binding molecule will be an antibody, for example a monoclonal antibody, which immunologically binds to the target compound at a specific determinant or epitope. The term "antibody" is used in the broadest sense and specifically covers monoclonal antibodies as well as antibody fragments (e.g., Fab, F(ab').sub.2, scFv, Fv diabodies and linear antibodies), so long as they exhibit the desired binding activity. For a review of sFv see Pluckthun (1994) The Pharmacology of Monoclonal Antibodies, Vol. 113. Rosenburg and Moore eds. Springer-Verlag, New York, pp. 269-315. Diabodies are described more fully in, for example, European patent 404097, international patent publication WO 93/11161 and Hollinger et al. (1993) Proc. Natl. Acad. Sci. USA 90: 6444-6448. Linear antibodies are described in Zapata et al. (1995) Protein Eng. 8(10): 1057-1062.

"Monoclonal antibodies" which are homogeneous populations of antibodies to a particular antigen, may be obtained by any technique that provides for the production of antibody molecules by continuous cell lines in culture. These include, but are not limited to the hybridoma technique of Kuhler and Milstein (1975), Nature, 256: 495-7; and U. S. Patent No. 4,376,110), the human B-cell hybridoma technique (Kosbor, et al. (1983), Immunology Today, 4: 72; Cote, et al. (1983), Proc. Natl. Acad. Sci. USA, 80: 2026-30), and the EBV-hybridoma technique (Cole, et al. (1985), in Monoclonal Antibodies And Cancer Therapy, Alan R. Liss, Inc., New York, pp. 77-96). The preparation of monoclonal antibodies specific for a target compound is also described in Harlow and Lane, eds. (1988) Antibodies - A Laboratory Manual. Cold Spring Harbor Laboratory, Chapter 6. Such antibodies may be of any immunoglobulin class including IgG, IgM, IgE, IgA, IgD and any subclass thereof. The hybridoma producing the mAb may be cultivated in vitro or in vivo. Production of high titers of mAbs in vivo makes this a very effective method of production.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. The monoclonal antibodies can include "chimeric" antibodies (U.S. Patent No. 4,816,567; and Morrison et al. (1984) Proc. Natl. Acad. Sci. USA, 81: 6851-6855) and humanized antibodies (Jones et al. (1986) Nature, 321: 522-525; Reichmann et al. (1988) Nature, 332: 323-329; Presta (1992) Curr. Op. Struct. Biol. 2: 593-596). A "chimeric" antibody is a molecule in which different portions are derived from different animal species, such as those having a variable region derived from a murine mAb and a human immunoglobulin constant region.

"Polyclonal antibodies" are heterogeneous populations of antibody molecules derived from the sera of animals immunized with an antigen, or an antigenic functional derivative thereof. For the production of polyclonal antibodies, host animals such as rabbits, mice and goats, may be immunized by injection with an antigen or hapten-carrier conjugate optionally supplemented with adjuvants.

Alternatively, techniques described for the production of single chain antibodies (U. S. Patent No. 4,946,778; Bird (1988), Science 242: 423-26; Huston, et al. (1988), Proc. Natl. Acad. Sci. USA, 85: 5879-83; and Ward, et al. (1989), Nature, 334: 544-46) can be adapted to produce gene-single chain antibodies. Single chain antibodies are typically formed by linking the heavy and light chain fragments of the Fv region via an amino acid bridge, resulting in a single chain polypeptide.

Antibody fragments that recognize specific epitopes may be generated by known techniques. For example, such fragments include but are not limited to: the F (ab') 2 fragments that can be produced by pepsin digestion of the antibody molecule and the Fab fragments that can be generated by reducing the disulfide bridges of the F (ab')2 fragments. Alternatively, Fab expression libraries may be constructed (Huse, et al. (1989), Science, 246: 1275-1281) to allow rapid and easy identification of monoclonal Fab fragments with the desired specificity.

The "sandwich" detection of a given target molecule may be carried out by using an identical polyclonal antibody as first binding ("capture") molecule and detection molecule. In this case, "identical" is defined as polyclonal antibodies from a single preparation, including antibodies against different binding sites of the target molecule. As the unspecific interaction of the polyclonal antibody with itself is minimized already during the genesis of the antibody, this approach may also be advantageous for minimization of assay background.

A variant of this approach is the use of an identical monoclonal antibody as capture and detection antibody if the target has several binding spots for this antibody, such as surface proteins in a virus shell, whereby in this application the virus shell would be the target.

The binding molecule may also be a lipocalin, such as the anticalins described in international patent publication WO 99/16873.

Alternatively, the binding molecule may also be a high-affinity nucleic acid ligand which binds to the target molecule, e.g. an aptamer. The term "nucleic acid ligand" as used herein means a nucleic acid, including naturally occurring and non-naturally occurring nucleic acids, having a specific binding affinity for the target molecule. Nucleic acid ligands may be identified and prepared using the SELEX method described in U.S. Patent No. 5,270,163; U.S. Patent No. 5,475,096; U.S. Patent. No. 5,496,938; WO 96/40991; and WO 97/38134, for example. The nucleic acid ligand may be DNA or RNA.

The binding molecule may also be a binding protein, receptor or extracellular domain (ECD) thereof capable of forming a binding complex with a ligand, typically a polypeptide or glycopeptide ligand.

The binding molecule may also be a phage-antibody. Antibodies and antibody fragments may be displayed on the surface of a filamentous bacteriophage as described in U.S. Patent No. 5,750,373, for example and the references cited therein. See also EP 844306; U.S. Patent No. 5,702,892; U.S. Patent No. 5,658,727; WO 97/09436; U.S. Patent No. 5,723,287; U.S. Patent No. 5,565,332; and U.S. Patent No. 5,733,743.

The "detection molecule" may be any of the above-mentioned binding molecules labelled with a DNA label or may be a high-affinity nucleic acid ligand, which binds to the target molecule. The detector antibody may be labeled with a DNA label using techniques known for use in conventional Immuno-PCR, for example, by cross-linking with Sulfo-SMCC (Pierce, Rockford, Ill.). Linking of detection molecule and DNA could also be carried out in a stepwise protocol, using e.g. a biotinylated detection molecule, streptavidin as a tetravalent, biotin-binding coupling molecule and subsequently a biotinylated nucleic acid.

In one embodiment, pre-formed antibody-DNA conjugates may be used as detection molecules as these conjugates significantly shorten the assay protocol and simplify assay handling, thus reducing possible error sources and increasing coupling efficiency.

In another embodiment, the detection molecule is formed by a binding molecule (1) which is bound by another binding molecule (2) coupled with an nucleic acid marker whereby the binding of the two binding molecules is achieved by using a binding molecule (2) which is specifically binding the binding molecule (1), e. g by using a target specific antibody form mouse as binding molecule (1) and a mouse specific binding molecule as binding molecule (2). In an alternative embodiment, binding molecule (1) is labelled with a specific marker (e.g. biotin) and the nucleic acid labelled binding molecule (2 specifically binds this marker.

Those skilled in the art will recognized that the non-limiting examples given above describing various forms of antibodies as binding and detection molecules can also be extended to other receptors such that recombinant, chimeric, hybrid, truncated etc., forms of non-antibody receptors can be used in the methods of the present invention.

The terms "polynucleotide" and "nucleic acid (molecule)" are used interchangeably to refer to polymeric forms of nucleotides of any length, including naturally occurring and non-naturally occurring nucleic acids. The polynucleotides may contain deoxyribonucleotides, ribonucleotides and/or their analogs. A "nucleic acid ligand" has a specific binding affinity for the target molecule. Methods for selection and preparation of nucleic acids are diverse al well described in standard biomolecular protocols. A typical way would be preparative PCR and chromatographic purification (Niemeyer et al. 1999) starting from existing template DNAs or stepwise synthesis of artificial nucleic acids.

Nucleotides may have any three-dimensional structure, and may perform any function, known or unknown. The term "nucleic acid molecule" includes single-, double-stranded and triple helical molecules. "Oligonucleotide" refers to polynucleotides of between 5 and about 100 nucleotides of single- or double-stranded nucleic acid, typically DNA.

Oligonucleotides are also known as oligomers or oligos and may be isolated from genes, or chemically synthesized by methods known in the art. A "primer" refers to an oligonucleotide, usually single-stranded, that provides a 3'-hydroxyl end for the initiation of enzyme-mediated nucleic acid synthesis.

The following are non-limiting embodiments of nucleic acids: a gene or gene fragment, exons, introns, mRNA, tRNA, rRNA, ribozymes, cDNA, recombinant polynucleotides, branched polynucleotides, plasmids, vectors, isolated DNA of any sequence, isolated RNA of any sequence, nucleic acid probes and primers. A nucleic acid molecule may also comprise modified nucleic acid molecules, such as methylated nucleic acid molecules and nucleic acid molecule analogs. Analogs of purines and pyrimidines are known in the art, and include, but are not limited to, aziridinylcytosine, 4-acetylcytosine, 5-fluorouracil, 5-bromouracil, 5-carboxymethylaminomethyl-2-thiouracil, 5-carboxymethyl-aminomethyluracil, inosine, N6-isopentenyladenine, 1-methyladenine, 1-methylpseudouracil, 1-methylguanine, 1-methylinosine, 2,2-dimethylguanine, 2-methyladenine, 2-methylguanine,3-methylcytosine,5-methylcytosine, pseudouracil, 5-pentylnyluracil and 2,6-diaminopurine. The use of uracil as a substitute for thymine in a deoxyribonucleic acid is also considered an analogous form of pyrimidine.

The term "nucleic acid marker" or "nucleic acid reporter" refers to a nucleic acid molecule that will produce a detection product of a predicted size or other selected characteristic when used with appropriately designed oligonucleotide primers in a nucleic acid amplification reaction, such as a PCR reaction. Skilled artisans will be familiar with the design of suitable oligonucleotide primers for PCR and programs are available, for example, over the Internet to facilitate this aspect of the invention (See, for example, http://bibiserv.techfak.uni-bielefeld.de/genefisher/). A nucleic acid marker may be linear or circular. In specific embodiments, the nucleic acid marker will comprise a predetermined, linear nucleic acid sequence with binding sites for selected primers located at or near each end. In a circular DNA nucleic acid molecule, the primers will be internal rather than at an end, and a single primer may be used, e. g. for Rolling Circle Amplification. Amplified DNA may be detected using any available method, including, but not limited to techniques such as real time PCR, SYBR Green staining, or ethidium bromide staining. In certain embodiments, the DNA sequence located between the primer binding sites comprises a "characteristic identification sequence" capable of being detected during the PCR reaction. Fluorescent signal generation may, for example, be sequence-specific (Molecular Beacons, Taq Man, Scorpions, fluorogenic primers, such as the LUX primers (Invitrogen (Carlsbad, CA.)) or mass dependent (SYBR Green, Ethidium Bromide). The examples provided are not meant to be an exhaustive list of possible nucleic acid detection schemes as those skilled in the art will be aware of alternative markers suitable for use in the methods of the present invention.

The term "characteristic identification sequence" refers to a nucleic acid sequence that can be specifically detected by virtue of hybridization to oligonucleotide or other nucleic acid that has been labeled with a detectable marker such as a radioisotope, a dye (such as a fluorescent dye), or other species that will be known in the art. In some embodiments, the characteristic identification sequence is capable of binding a "molecular beacon" probe. The term "molecular beacon" refers to oligonucleotides such as those sold by Operon Technologies (Alameda, CA, USA) and Synthetic Genetics (San Diego, CA, USA). (See also, Tyagi and Kramer (1996), Nat. Biotechnol, 14 : 303-308; and Tyagi et al. (2000), Nat Biotechnol, 18 : 1191-96). In another specific embodiment, the identification sequence is capable of binding a Scorpion. "Scorpions" are bifunctional molecules containing a PCR primer covalently linked to a probe. The fluorophore in the probe interacts with a quencher which reduces fluorescence. During a PCR reaction the fluorophore and quencher are separated which leads to an increase in light output from the reaction tube. Scorpions are sold by DxS Ltd. (Manchester, UK). As noted herein, a signal can be generated using a variety of techniques and reagents.

In connection with the sample dilution buffer "nucleic acid" or "nucleic acid molecule" means all nucleic acids not used as a marker nucleic acid in the detection molecule. In a specific embodiment, a homogenous mixture of different DNA sequences such as a pool of DNA fragments in preparations e.g. from fish sperm (supplied e.g. as DNA MB grade, Roche) is used.

"Peptide" generally refers to a short chain of amino acids linked by peptide bonds. Typically peptides comprise amino acid chains of about 2-100, more typically about 4-50, and most commonly about 6-20 amino acids. "Polypeptide" generally refers to individual straight or branched chain sequences of amino acids that are typically longer than peptides. "Polypeptides" usually comprise at least about 100 to 1000 amino acids in length, more typically at least about 150 to 600 amino acids, and frequently at least about 200 to about 500 amino acids. "Proteins" include single polypeptides as well as complexes of multiple polypeptide chains, which may be the same or different.

Multiple chains in a protein may be characterized by secondary, tertiary and quaternary structure as well as the primary amino acid sequence structure, may be held together, for example, by disulfide bonds, and may include post-synthetic modifications such as, without limitation, glycosylation, phosphorylation, truncations or other processing.

Antibodies such as IgG proteins, for example, are typically comprised of four polypeptide chains (i. e., two heavy and two light chains) that are held together by disulfide bonds. Furthermore, proteins may include additional components such associated metals (e. g., iron, copper and sulfur), or other moieties. The definitions of peptides, polypeptides and proteins includes, without limitation, biologically active and inactive forms; denatured and native forms; as well as variant, modified, truncated, hybrid, and chimeric forms thereof. The peptides, polypeptides and proteins of the present invention may be derived from any source or by any method, including, but not limited to extraction from naturally occurring tissues or other materials; recombinant production in host organisms such as bacteria, fungi, plant, insect or animal cells; and chemical synthesis using methods that will be well known to the skilled artisan.

When used in connection with the sample dilution buffer, the terms "protein" and "peptide" include all proteins and/or peptides not used as target or binding molecule in a given assay. In one embodiment, a single, well characterized protein such as e.g. bovine serum albumin, ovalbumin or casein is used. In another embodiment, a mixture of different proteins, such as skim milk powder for molecular biology applications or fetal bovine serum is applied to include more interaction potential. These proteins are not intended to be recognized by any of the binding molecules or reagents used in the IPCR assays. This unrecognized protein therefore allows the immunological blocking of non-specific binding events by molecules or compounds which might be present in the sample.

"Detergents" are typically non-ionic detergents. Examples are detergents from the group of Dodecylpoly (ethylenealycolether)ₘ, wherein m is an integer of 5 to 40, 1-O-n-Octyl-α-D-glucopyranoside (n-Octylglucoside), Alkylphenolpoly (ethyleneglycol- ether)ₘ, wherein m is an integer of 5 to 40, for example m=11 (Nonidet P40), 1-O-n-Dodecyl-α-D-glucopyranosyl (1→4) alpha-D-glucopyranoside, Dodecylpoly-(ethyleneglycolether)ₘ, wherein m is an integer of 5 to 40, for example m = 23 (Brij35), Poly (oxyethylene) (20) - sorbitane mono fatty acid ester, preferably selected from Poly(oxyethylene) (20)-sorbitane monooleate (Tween 80), Poly(oxyethylene) (20)-sorbitane monolaurate (Tween 20), Poly(oxyethylene) (20)-sorbitanemonopalmitat (Tween 40), and Poly(oxyethylene) (20)-sorbitane monostearate), and Octylphenolpoly (ethyleneglycolether)ₘ, wherein m is an integer of 5 to 40, for example m=10 (TritonX-100).

"Chelate-building stabilizers" or "chelating agents" are included as many sample-destabilizing enzymes require small molecule co-factors such as e.g. Mg²⁺ which can be captured by chelate-building compounds, thereby effectively inactivating the interfering enzymes. Typical chelate builders include EDTA, crone ethers etc.

"Salts" in connection with the sample dilution buffer of the invention encompasses salts and buffer components for maintaining pH and salt concentration. As salt solutions, aqueous solutions of salts from the following groups are chosen: NaCl, KCl, NH₄Cl. In a specific embodiment, the buffer compounds are selected from the group Tris (Tris (hydroxymethyl)-aminomethane, Pipes (Piperazine-1,4-bis-2-ethane sulfonic acid), Mes (4-Morpholino ethane sulfonic acid), Hepes (4-(2-hydroxyethyl)-1-piperazine-ethane sulfonic acid) and phosphates.

The term "solid support" refers any solid phase that can be used to immobilize e.g., an analyte, an antibody or a complex. Suitable solid supports will be well known in the art and include the walls of wells of a reaction tray, such as a microtiter plate, the walls of test tubes, polystyrene beads, paramagnetic or non-magnetic beads, nitrocellulose membranes, nylon membranes, microparticles such as latex particles, and sheep (or other animal) red blood cells. Typical materials for solid supports include, but are not limited to, polyvinyl chloride (PVC), polystyrene, cellulose, nylon, latex and derivatives thereof. Further, the solid support may be coated, derivatized or otherwise modified to promote adhesion of the desired molecules (e. g., analytes) and/or to deter non-specific binding or other undesired interactions. The choice of a specific "solid phase" is usually not critical and can be selected by one skilled in the art depending on the assay employed. Thus, latex particles, microparticles, paramagnetic or non-magnetic beads, membranes, plastic tubes, walls of microtiter wells, glass or silicon chips, and red blood cells are all suitable sold supports. Conveniently, the solid support can be selected to accommodate various detection methods. For example, 96 or 384 well plates can be used for assays that will be automated, for example by robotic workstations, and/or those that will be detected using, for example, a plate reader. For methods of the present invention that may involve an autoradiographic or chemiluminescent detection step utilizing a film-based visualization, the solid support may be a thin membrane, such as a nitrocellulose or nylon membrane. According to one embodiment of the invention in which sandwich immunoassays are performed, the walls of the wells of a reaction tray are typically employed. In alternative embodiments of the instant invention, paramagnetic beads may be used as a solid support. Suitable methods for immobilizing molecules on solid phases include ionic, hydrophobic, covalent interactions and the like, and combinations thereof. However, the method of immobilization is not typically important, and may involve uncharacterized adsorption mechanisms. A "solid support" as used herein, may thus refer to any material which is insoluble, or can be made insoluble by a subsequent reaction. The solid support can be chosen for its intrinsic ability to attract and immobilize a capture reagent. Alternatively, the solid phase can retain an additional receptor which has the ability to attract and immobilize a capture reagent. The additional receptor may include a substance that is oppositely charged with respect to either the capture reagent itself or to a charged substance conjugated to the capture reagent. In yet another embodiment of the invention, an additional receptor molecule can be any specific binding member which is immobilized upon (attached to) the solid phase and which has the ability to immobilize a capture reagent through a specific binding reaction. The additional receptor molecule enables indirect immobilization of the capture reagent to a solid phase before or during the performance of the assay. The solid phase thus can be a plastic, derivatized plastic, paramagnetic or non-magnetic metal, glass or silicon surface of a test tube, microtiter well, sheet, bead, microparticle, chip, or other configurations known to those of ordinary skill in the art.

The terms "contacting" or "incubating" as used interchangeably herein refer generally to providing access of one component, reagent, analyte or sample to another. For example, contacting can involve mixing a solution comprising a non-nucleic acid receptor with a sample. The solution comprising one component, reagent, analyte or sample may also comprise another component or reagent, such as dimethyl sulfoxide (DMSO) or a detergent, which facilitates mixing, interaction, uptake, or other physical or chemical phenomenon advantageous to the contact between components, reagents, analytes and/or samples. In one embodiment of the invention, contacting involves adding a solution comprising a non-nucleic acid receptor to a sample utilizing a delivery apparatus, such as a pipette- based device or syringe-based device.

The term "detecting" as used herein refers to any method of verifying the presence of a given molecule. The techniques used to accomplish this may include, but are not limited to, PCR, sequencing, PCR sequencing, molecular beacon technology, scorpions technology, hybridization, and hybridization followed by PCR. Examples of reagents which might be used for detection include, but are not limited to, radio-labeled and fluorescently probes and dyes, such as DNA intercalating dyes.

The term "hapten" as used herein, refers to a small proteinaceous or non-protein antigenic determinant which is capable of being recognized by an antibody. Typically, haptens do not elicit antibody formation in an animal unless part of a larger species. For example, small peptide haptens are frequently coupled to a carrier protein such as keyhole limpet hemocyanin in order to generate an anti-hapten antibody response.

"Antigens" are macromolecules capable of generating an antibody response in an animal and being recognized by the resulting antibody. Both antigens and haptens comprise at least one antigenic determinant or "epitope", which is the region of the antigen or hapten which binds to the antibody. Typically, the epitope on a hapten is the entire molecule.

The term "conjugate" as used herein refers to two or more molecules which have been covalently attached, or otherwise linked together. In one embodiment, a nucleic acid conjugate is generated by covalently linking the nucleic acid to a polypeptide. In a certain embodiment of the invention, a nucleic acid reporter sequence and a detection molecule are covalently or non-covalently attached via a linking group to form a conjugate. In a particular embodiment, the conjugate comprises, consists essentially of or consists of a biotinylated DNA molecule coupled via a streptavidin molecule to a target-specific biotinylated antibody. Such an conjugate may be an oligomeric conjugate, i.e. comprise more than one reporter nucleic acid sequence and/or more than one detection molecule, and/or, if present, more than one linker molecules.

### Preferred embodiments

The instant invention is based on the inventor's surprising finding that the addition of nucleic acid molecules to a sample that is to be assayed in an Immuno-PCR can reduce the background and thus enhance the signal-to-background ratio. According to the invented method the nucleic acid molecules are contained in a suitable buffer composition, for example a sample dilution buffer that may contain other components, such as proteins, detergents, buffer substances, salts and chelating agents. The nucleic acid molecule(s) are added to the sample prior to the IPCR assay procedure that may involve in a first step immobilizing the analyte on a solid support, such as a microtiter plate well, by means of a (immobilized) capture molecule or direct immobilization, and/or the coupling of the analyte to an analyte-specific binding molecule, such as a reporter antibody or IPCR conjugate.

This newly discovered principle allows for an improved Immuno-PCR performance, which is readily available, cost-effective and avoids laborious and error-prone sample purification protocols.

Due to the mandatory use of antibody-nucleic acid conjugates, Immuno-PCR assays in contrast to other immunoassays include nucleic acids. As nucleic acid molecules are normally of no relevance for ELISA and other known immunoassays, they are of no relevance for common buffer compositions used in the art for immunoassays and sample dilution. Nucleic acids were up to date also not included in sample dilution buffers as used in the application of the aforementioned IPCR assays (Adler et al., supra).

However, in blocking solutions used for coating the surface of Immuno-PCR vessels against unspecific interactions as well as in buffers used for dilution of the antibody-DNA conjugate, DNA is typically added with the intention to minimize possible unspecific interactions of the DNA-marker attached to the detection antibody with the surface of the vessel or other compounds present in the assay (Sano et al., supra; Zhou et al., supra). Nevertheless, a number of IPCR assays were reported which revealed good functionality without additional DNA in the blocking solution (Henterich et al. (2003). "Assay of gliadin by real-time immunopolymerase chain reaction." Nahrung 47(5): 345-8, McKie et al. (2002). "A quantitative immuno-PCR assay for the detection of mumps-specific IgG." J. Immunol. Methods 270(1): 135-41, Case et al. (1999) "Enhanced ultrasensitive detection of structurally diverse antigens using a single immuno-PCR assay protocol." J. Immunol. Methods 2230(1): 93-1061, while no quantitative information about the positive effects of the DNA is given in other sources. It is therefore obvious for those skilled in the art that additional DNA in these buffers/solutions is no critical part for the performance of the IPCR.

Without any positive effect of the DNA in the blocking buffer or the storage buffer of the antibody-DNA conjugate being reported to date, it is not surprising that the addition of DNA to the sample incubation step in IPCR has not been contemplated before, particularly in view of the fact that no antibody-DNA conjugates are present at this stage of the assay.

DNA in the sample dilution buffer was expected to have no influence on assay performance because all potentially interfering compounds were typically washed away previous to the incubation with the antibody-DNA conjugate. Moreover, it was known to the experienced user of the Immuno-PCR technique that sample-endemic DNA also has no influence on the performance of the PCR due to the washing steps of the sandwich technique previous to PCR.

Furthermore, if DNA is used in the blocking solution, it is already bound to the IPCR vessel surface so that the immobilized DNA used for blocking is present during the incubation of the sample and the performance of the PCR. Accordingly, it was expected that the addition of a small amount of this DNA during the sample incubation step followed by subsequent washing should have no additional effect whatsoever on the subsequent coupling of the antibody-DNA conjugate and/or the performance of the PCR in relation to the already large amount of immobilized DNA which is present in the wells due to the blocking step.

It was therefore very surprising to find a positive influence of added DNA to the sample prior to the Immuno-PCR assay.

Completely unexpected was the observation that the influence of the DNA was not an isolated effect but revealed a strong interaction with proteins also added to the sample dilution buffer.

Therefore, a novel way to positively influence the specific binding of target compounds in IPCR due to new cooperative interactions of proteins and DNA was found. With the addition of DNA and proteins to the sample, it is possible to strongly increase the signal-to-background ratio of the Immuno-PCR assay for the specific detection of an analyte and thereby significantly increase IPCR performance in biological matrices.

Consequently, in a first aspect the present invention is directed to a method for the improvement of a given Immuno-PCR ("IPCR") assay of a biological sample, wherein the method comprises the dilution of the sample with a buffer composition containing one or more nucleic acid molecules, for example DNA molecules wherein the concentration of the nucleic acid molecules in the buffer is between about 0.01 mg/ml and about 10 mg/ml. Optionally, in order to further improve the buffer effect on Immuno-PCR performance, the buffer can additionally contain one or more proteins and/or peptides. Without wishing to be bound to any particular theory, it is assumed that the interaction of these components with each other and the sample material accounts for the increase of the performance of the IPCR assay. The application of this buffer by adequate dilution of the matrix sample will provide a quantitative method for detecting the absence, the presence and/or the amount of a target compound in a biological sample where the method has compared to conventional methods an improved sensitivity, an improved dynamic range, an improved assay precision, improved resistance to contaminations of the sample and where overall assay handling is simplified.

Due to the improved binding in the presence of the new sample dilution buffer, the signal-to-background ratio in assay read-out is significantly improved, thereby also enhancing the sensitivity of the overall assay.

The invention, therefore, provides improvements in quantitation and sensitivity over conventional Immuno-PCR assays which are very liable to background effects e.g. caused by the detection of an antigen in a complex biological matrix. Thus, the method of the invention represents an improvement over conventional Immuno-PCR in which no special sample dilution buffer is applied to moderate and improve the binding of the several compounds of the assay, especially the specific binding of the target compound to the capture molecule. Furthermore, the invented method also allows for a new kind of IPCR application by direct immobilization of target compounds to a binding surface from complex biological matrix due to the presence of the sample dilution buffer.

The invention also provides improvements over conventional ELISA assays in sensitivity.

In a first embodiment, the instant invention thus features a method for determining the presence and/or amount of an analyte in a sample by performing an Immuno-PCR assay, wherein the method includes the use of a sample dilution buffer for dilution of the sample, wherein the buffer solution comprises one or more nucleic acid molecules in a concentration of between about 0.01 mg/ml and about 10 mg/ml and optionally, one or more protein and/or peptide molecules.

When performing the method of the invention, the presence of an analyte in a sample which may contain the analyte can be detected and may be quantitated by exposing the sample to a binding molecule capable of binding the analyte to form a specific complex of binding molecule and analyte.

Specific binding is achieved by either a single binding molecule as part of the detection molecule, described below, or by using two specific binding molecules, a first binding molecule as a capture and a second binding molecule as a detection molecule.

For sandwich detection, a first binding ("capture") molecule may be attached to a solid support before or after forming the binding complex with the target molecule, that is to say the analyte. Specific capture molecules, e.g. antibodies or aptamers, are prepared and purified using conventional preparation and separation techniques. The capture molecules are then attached to solid supports using passive absorbance or other conventional (e.g., chemical) techniques for attaching proteins to solid supports. The complex formed by the binding of the capture molecule and the target molecule or a complex from the detection molecule and the target molecule or a ternary complex of capture molecule, target and detection molecule may be formed in solution phase and immobilized on the solid support. In a specific embodiment, the solid support is coated with one member of a known binding pair, e.g. avidin, streptavidin, or a biotin-binding antibody, and the capture molecule is labeled with the other member of the binding pair, e.g. biotin. The biotin-labelled complex of biotinylated capture molecule and target molecule or a ternary complex of biotinylated capture molecule, target and detection molecule may be formed in solution phase and captured by the streptavidin coated support. Alternatively, the capture molecule is immobilized on the solid surface previous to the exposition to the sample and, upon incubation with the sample forms a complex with the target molecule, or, in case the sample has been pre-incubated with the detection molecule or the detection molecule is simultaneously present upon incubation with the immobilized capture molecule, with a complex from the detection molecule and the target molecule.

Another option for the coupling reaction is the use of surfaces coated with a species-specific antibody in combination with first binding molecules (capture molecules) which are target specific antibodies from this species.

Prior to the above-described complex formation step, the sample can be diluted in the sample dilution buffer containing one or more nucleic acid molecules in a dilution range of 0.1:1 - 1: 100, 0.5:1 - 1:50, 1:1 - 1:10, or 1:1 - 1:3. Alternatively, the sample is diluted with the nucleic acid-containing buffer during the complex formation between target molecule and capture and/or detection molecule. In this case, the binding molecule, i.e. either the detection or the capture molecule, is diluted in the sample dilution buffer, thereby combining sample dilution and binding in a single step.

If the target molecule is directly immobilized on the solid support (direct assay format), the sample is directly exposed to an adsorbing support surface (e.g. polycarbonate, polystyrene, etc.). In case the sample consists of biological material, typically all compounds present in the biological material will bind against the support surface. Therefore, if the amount of target compound in the sample material is very small in comparison to other compounds in the sample, the other compounds will inhibit binding of the target compound. To allow the binding of the target compound from a biological matrix it may therefore be advantageous to use a high dilution of the sample in sample dilution buffer during the exposition to the support surface. For direct IPCR dilution ratios of 1:10 - 1:500, particularly 1:10-1:100 can be used. Similar to the above-described sandwich assay format, this dilution of the sample with the sample dilution can also be performed previous to the exposition of the sample to the support surface.

When carrying out the invented method, the coupling of the first binding molecule to the support in the sandwich assay or the direct coupling of the sample against the surface is typically carried out overnight at 4°C. If an additional surface-bound coupling compound is used for immobilizing the first binding molecule, e.g. for immobilization of a biotinylated binding molecule an a surface coated with streptavidin, this coupling compound is incubated overnight, respectively, while the actual coupling of the first binding molecule and the immobilized coupling compound is carried out in much shorter time, for example in 30 min at ambient temperature.

Any suitable solid support is useful in the method of the present invention. Suitable solid supports include membranes, charged paper, nylon, beads, polystyrene ELISA plates, PCR tubes (Numata et al, 1997), V-bottom polycarbonate plates (Chang et al, 1997), etc. Suitable membranes include nitrocellulose membranes and polyvinylidine difluoride membranes. In a specific embodiment, the capture molecule is bound to a polymer bead, tube or plate, for example a conventional polycarbonate plate.

In one form of this embodiment, the support is a either capture molecule or streptavidin coated vessel compatible with PCR cyclers and ELISA equipment, e.g. a 8 or 12 well stripe of PCR tubes or a 96 or 384 well PCR plate. This procedure eliminates the need of a transfer step from conventional ELISA vessels to PCR tubes as required for signal amplification and allow for the routine use of typically laboratory equipment compatible with the geometry of ELISA applications, such as automatic plate washers, multichannel pipettes, etc.

To minimize non-specific binding of the target molecule to a solid support in a sandwich assay and the non-specific binding of the binding molecules in all types of assays, the solid support can be treated with a blocking solution to block non-specific binding sites prior to exposing the sample to the capture molecule in a sandwich assay or subsequent to the immobilization of the target against the surface in a direct assay.

Common blocking agents include dilute protein solutions (about 3-5%), for example bovine serum albumin (BSA) or milk powder, and DNA preparations, such as DNA fragments from fish sperm. In a specific embodiment, blocking is carried out overnight (8 - 48 h) at 4°C according to methods known to those skilled in the art to ensure a homogenous and sufficient blocking. The blocking solution is then generally washed from the solid support to remove remaining blocking agent.

In addition to the first exposure of the sample either to the first binding molecule or the surface, the target is coupled to the detection molecule. The coupling with the detection molecule can be carried out subsequent to the coupling with the first binding molecule, separated by a washing step, or in another aspect of the invention, simultaneously with the coupling against the first binding molecule. In another embodiment, labelled first binding molecule and the sample are incubated simultaneously in a vessel able to bind the label of the labelled first binding molecule. In both cases, the binding molecule either the capture and/or the detection molecule is diluted in the sample dilution buffer, thereby combining sample dilution and binding in a single step.

In a specific embodiment, the incubation time of the target with the detection conjugate is between 25 min and 12 h, particularly 30 min at room temperature with orbital shaking.

Ordinary systematic optimization of assay parameters such as concentrations of the binding molecules or incubation time lies within the knowledge and skill of the practitioner in this field and will generally be performed for each different target / binding molecule / matrix combination.

During the incubation of the detection molecule, a ternary complex consisting of first binding molecule, target, and detection molecule is formed in case of the sandwich assay. In case of the direct assay, a binary complex of target and detection molecule is formed.

The detection molecule is generally dissolved in an aqueous solution, for example an aqueous buffer solution. Suitable buffers are those well known in the art for buffering antibody and nucleic acid ligand molecules, and include known buffers used in conventional ELISA, and Immuno-PCR assays. The buffer used for the solution of the detection molecule can consist of the same components as the sample dilution buffer, thus ensuring a complimentary set of interaction potentialities during the complete assay.

In another embodiment, several different detection molecules coupled with different nucleic acid marker sequences are incubated simultaneously for a multiplex assay. This multiplex assay enables the user to perform a parallel testing for different target molecules in a single assay, whereby in a particular embodiment different detection probes in real-time PCR are used for the specific detection of each marker sequence. In one embodiment of this approach, the number of targets which are detected simultaneously is 2-5.

For washing steps to remove non-bound compounds, wash buffer including detergents, chelate builders and salt at a pH of 7.4 can be used. Washing is typically carried out by using a multichannel pipetting device.

In one embodiment, washing is carried out by a semi-automatic or fully automatic microplate washing device, thereby enabling the user to perform washing in a closed environment, especially well suited for the analysis of potentially hazardous target compounds and/or biological sample materials. By using automatic washing devices, residual waste buffer form washing will be collected and inactivated, thereby ensuring the correct disposal of potentially hazardous materials.

After a final washing step to remove unbound compounds and the addition of a reagent mixture for signal amplification of the nucleic acid marker in the detection molecule, the assay is ready for read-out.

According to the afore-mentioned methods, nucleic acid molecules and, optionally, additional peptide and/or protein molecules are added to the sample, e.g. in form of an aqueous buffer solution, as this reduces the background signals due to matrix effects and unspecific bindings, thereby increasing the signal-to-background ratio. Furthermore, the dilution of the sample allows the laboratory use to work with a minuscule amount of sample material, which is especially important if only small quantities of the sample are available, e.g. in detecting a target compound in body fluids of small animal. The total assay volume may be as low as 30 µl / well, thus for a typical 1:5 dilution in sample dilution buffer, only 6 µl of the actual sample would be required.

In one embodiment of the invention, the sample of interest is therefore diluted with a sample dilution buffer containing one or more nucleic acid molecules and, optionally, one or more additional components that are selected from the group of buffer compounds, proteins, chelating agents and detergents, so that coupling of the target analyte and either the specific binding molecule or the binding surface is carried out in the presence of said sample dilution buffer.

The exact composition of the buffer can be adapted to the biological matrix by systematic variation of the concentration of these compounds in relation to their influence of the signal-to-background ratio observed in the determination of the detection of the target.

In a specific embodiment of the invention, the above-described nucleic acid and, optionally, peptide/protein containing sample dilution buffer, may further contain additional buffer components, such as detergents, chelating agents and salts.

In one embodiment, the ratio of these buffer components as well as the ratio of the dilution of the sample in the sample dilution buffer is subsequently evaluated in a series of systematic parameter variation experiments for each combination of target molecule / binding molecule(s) / sample material regarding best performance as obvious from the signal-to-backaround ratio obtained for the comparison of spiked matrix samples with and without the target compound.

In another embodiment, a standard sample dilution buffer is used for each type of matrix (e.g. serum or cell lysate) and only the dilution ratio and the amount of DNA and protein is permutated.

In one embodiment of the invention, the concentration range of the nucleic acid, for example, DNA in the sample dilution buffer is from about 0.1 - 10 mg/ml DNA. If present, the concentration of protein and/or peptides in the sample dilution buffer ranges from about 0.1 - 10% by weight. If the buffer contains a detergent, the detergent concentration may lie in the range of 0.01 - 1 % (v/v). Chelating agents may be contained in the buffer in a concentration range of 0.5 - 50 mM. In a specific embodiment, the sample dilution buffer has a salt concentration of 1 - 200 mM and a pH value of 7-8. In one embodiment the pH is a physiological pH value of 7.4.

An exemplary buffer may, without limitation, contain a salt, such as Tuis-HCl (Trishydroxymethylaminomethane-HCl), a chelating agent, such as EDTA, a detergent, such as Tween 20, proteins, such as those contained in skim milk powder, and nucleic acid, such as DNA.

Exemplary concentrations of the different constituents in an aqueous sample dilution buffer are 20 mM Tris/HCl pH 7.4, 5 mM EDTA, 0.1% Tween 20, 2.5 % Skim milk powder, 0,5 mg/ml DNA.

The object of the present invention is also solved by using a concentrate of the sample dilution buffer of the present invention as described before, for example a 2- to 10-fold concentrate or a 3- to 5-fold concentrate.

A kit for detecting the presence of a selected analyte in a sample by the methods of the invention comprises at least one container means having disposed therein the sample dilution buffer according to the instant invention. The kit may further comprise one or more additional container means having disposed therein a solid support, binding molecules (in case of sandwich assays either bound to the solid support or modified for binding against a solid support which is coated with an appropriate coupling surface), detection molecule(s), wash buffers, reagents capable of amplifying the reporter nucleic acid sequence, reagents for the detection of the amplified nucleic acid amplicons, a calibration solution of the target compound, and positive and negative controls.

The kit may further comprise instructions for use. The kit, if intended for diagnostic use, may also include notification of an approval by the regulatory authorities, for example the FDA or EMEA, and instructions therefor.

One skilled in the art will readily recognize that the sample dilution buffer described in the present invention can readily be incorporated into one of the established kit formats that are well known in the art.

In the kit the sample dilution buffer composition may be in solid, for example lyophilized form for dissolving in water prior to use or may be provided as an aqueous solution. The detection molecule can comprise an analyte-binding molecule, a reporter nucleic acid sequence, and, optionally, a linker molecule either separately or already in form of a ready-to-use Immuno-PCR conjugate. If the components of the detection molecule are provided separately, the kit may further comprise means for forming the Immuno-PCR conjugate, such as buffers, coupling reagents, and instructions.

The reagents for signal amplification may include a ready-to-use PCR mix, or the amplification primers, PCR buffers and enzymes, as well as the detection means, such as labeled nucleic acid probes or dyes, such as ethidium bromide or SYBR Green, may be provided in separate container means.

In a specific embodiment, the nucleic acid-containing sample dilution buffer will be part of a complete kit of IPCR reagents, therefore enabling the user to perform the optimized assay with all complementary reagents and materials.

Detection of the analyte in a sample is achieved by observing a detectable signal from a detectable nucleic acid probe attached to a target-specific detection molecule which is further added to the target, either during exposition of the sample to the first binding molecule (capture molecule) or subsequent to binding of the target to the first binding molecule or a binding surface.

In case of subsequent incubation steps, these steps can be separated by an additional washing step to remove unbound molecules.

In one specific embodiment of the invention, the detectable nucleic acid probe is attached to the target-specific detection molecule previous to exposure of the detection-molecule to the target. Attachment is achieved either by covalent crosslinking or by supramolecular interaction. For covalent crosslinkers, heterobifunctional crosslinkers are one specific example; in case of supramolecular coupling, the aforementioned biotin-binding system may be used.

In another embodiment, coupling of detection molecules and the detectable nucleic acid probe is performed in a series of subsequent steps by using an intermediate coupling molecule.

The detection molecule may be an antibody labeled with a detectable nucleic acid probe, for example a DNA reporter sequence, wherein the labelling may, for example, have been achieved by direct covalent coupling or, in case the antibody and/or the DNA are biotinylated, via a streptavidin, avidin or fusion protein comprising streptavidin or avidin functioning as a linker.

Detection and, in a specific embodiment, also quantitation of the analyte is achieved using the detectable nucleic acid probe attached to the detection molecule.

In one embodiment, the detectable nucleic acid probe is a DNA molecule and detection is achieved by amplification of the DNA molecule in a PCR step. This can include simultaneous detection of the amplified DNA in a real-time PCR.

When the detection molecule is a DNA labeled antibody, the nucleic acid moiety may be directly subjected to PCR amplification for quantification of the marker.

The elevated temperatures which occur during standard PCR amplification reactions are sufficient to release the detector molecule from the detection complex for amplification.

In another embodiment, the DNA marker is detected by real time PCR, carried out in a commercially available instrument. Real-time PCR amplification is performed in the presence of a fluorescent-labelled probe which specifically binds to the amplified PCR product, for example a dual labelled primer including a fluorescent moiety quenched by another label which is in spatial proximity to the fluorescent label as long as the primer is not incorporated in an amplification product and separated from each other due to elongation of the primer during amplification.

In another embodiment, a non-primer detectable probe which specifically binds the PCR amplification product is used. The probe may include a covalently bonded reporter dye at the 5'-end and a downstream quencher dye at the 3'-end, which allow fluorescent resonance energy transfer (FRET).

Detection of the amplified PCR product may be carried out after each amplification cycle, as the amount of PCR product is at every stage of the amplification reaction proportional to the initial number of template copies. The number of template copies can be calculated by means of the detected fluorescence of the reporter dye. In an intact probe the fluorescence is quenched due to the close proximity of the reporter dye and quencher dye. During PCR, the nuclease activity of the DNA polymerase cleaves the probe in the 5'-3' direction and thus separates the reporter dye from the quencher dye. Because reporter and quencher dye are then no longer in close proximity to each other, the fluorescence of the reporter dye is increased. The increase in fluorescence is measured and is directly proportional to the amplification during PCR. See Heid et al. (1996), "Real time quantitative PCR" Genome Research 6(10):986-994. This detection system is now commercially available as the TaqMan.RTM PCR system from Perkin-Elmer, which allows real time PCR detection.

In an alternative embodiment, the reporter dye and quencher dye may be located on two separate probes which hybridize to the amplified PCR detector molecule in adjacent locations sufficiently close to allow the quencher dye to quench the fluorescence signal of the reporter dye (Rasmussen et al. (1998), "Quantitative PCR by continuous fluorescence monitoring of a double strand DNA specific binding dye" Biochemica 2:8-15). As with the detection system described above, the 5'-3' nuclease activity of the polymerase cleaves the one dye from the probe containing it, separating the reporter dye from the quencher dye located on the adjacent probe preventing quenching of the reporter dye. As in the embodiment described above, detection of the PCR product is by measurement of the increase in fluorescence of the reporter dye.

In other embodiments of this invention, other real time PCR detection strategies may be used, including known techniques such as intercalating dyes (ethidium bromide) and other double stranded DNA binding dyes used for detection (e.g. SYBR green, FMC Bioproducts), dual fluorescent probes (Wittwer et al. (1977) BioTechniques 22: 130-138 and Wittwer et al. (1997) BioTechniques 22: 176-181) and panhandle fluorescent probes (i.e. molecular beacons; Tyagi and Kramer (1996) Nature Biotechnology 14: 303-308). Although intercalating dyes and double stranded DNA binding dyes permit quantitation of PCR product accumulation in real time applications, they suffer from a lack of specificity, detecting primer dimer and any non-specific amplification product. Careful sample preparation and handling, as well as careful primer design, using known techniques are necessary to minimize the presence of matrix and contaminant DNA and to prevent primer dimer formation. Appropriate PCR instrument analysis software and melting temperature analysis permit a means to extract specificity (Ririe, K., et al. (1977) Anal. Biochem. 245: 154-160) and may be used with these embodiments.

In still another embodiment of this invention, the scorpions reaction is used as a real time PCR detection method. Scorpions are bi-functional molecules containing a PCR primer covalently linked to a probe. The fluorophore in the probe interacts with a quencher which reduces fluorescence. During the PCR reaction the primer binds to the template and is elongated by the polymerase. Once the elongation reaction is completed and primer and template are separated in the denaturation step, the elongated primer sequence can interact intramolecularly with the probe sequence in the next annealing step. The binding of the probe to the elongated primer sequence prevents interaction of the probe-bound fluorophore with the quencher, which leads to an increase in light output from the reaction tube. Currently, there are two formats for Scorpions, the bimolecular Scorpion format and the unimolecular format. In the bimolecular format the quencher is bound to a separate nucleic acid molecule which is complementary to the probe sequence, whereas in the unimolecular format both, fluorophore and quencher, are attached to the same molecule, and an integral stem loop sequence is used to bring the quencher close to the fluorophore. The scorpions technique is described more fully in Whitcombe et al. (1999), Detection of PCR products using self-probing amplicons and fluorescence, Nature Biotech 17, pages 804-807. This detection system is now commercially available as the scorpions system from DxS Ltd. (Manchester, UK).

The design of primers for the amplification reaction and nucleic acid probes is well-established in the art and thus routine practice for the skilled person. Suitable fluorescent reporter dyes are also known and commercially available, and include, without limitation 6-carboxy-fluorescein (FAM), tetrachloro-6-carboxy-fluorescein (TET), 2,7-dimethoxy-4,5-dichloro-6-carboxy-fluorescein (JOE) and hexachloro-6-carboxy-fluorescein (HEX). Another suitable reporter dye is 6-carboxy-tetramethyl-rhodamine (TAMRA).

The Immuno-PCR method of the invention has a dynamic range which allows the detection of target molecules at amounts from about 1000 molecules (approx. 1.6. zeptomole) to about 6x10¹³ molecules (approx. 10 pmole) in biological sample materiaL. The method can be used to detect target molecules of a typical size of 10,000 - 200,000 g/mol at concentrations in the range of about 0.01 pg/ml to about 100,000 pg/ml.

The method of the present invention is useful for the detection of target compounds in impure sample material, e.g. in biological matrices. The method expands the range of applications accessible with conventional ELISA or IPCR technology by increasing robustness and sensitivity of the methods. The additional dilution step reduces the amount of required sample for each analysis, minimizes the unspecific binding of matrix compounds and neutralizes inhibiting compounds.

Without being limited to these applications, the invented assay method may be used for the detection of marker proteins for diseases, viral proteins, toxins, new drugs, specific antibodies or modified proteins in sample material such as body fluids, tissue, food samples, environmental samples, etc.

Due to the enhanced robustness of the assay method, the stressful sample preparation and purification previous to an assay from biological matrix is significantly simplified.

Using the method of the invention, detector molecules containing a nucleic acid moiety can be directly detected and quantitated across at least five logarithmic concentrations in biological matrices, to as low as approx. thousand molecules. Since amplification and detection occurs directly in PCR vessels in some embodiments, there is no need for post-PCR analysis and manipulations and the risk of cross contamination between assays associated with these methods is minimized.

### Examples

The present inventions will be explained in more detail in the following examples. However, the examples are only used for illustration and do not limit the scope of the present invention.

### Example 1: Sandwich detection of several antigens from different biological matrices including the application of different sample dilution buffers

Microtiter modules ("TopYield", cat.-no. 248909, NUNC, Wiesbaden, Germany) were coated with 30µl / well of target-antigen specific capture antibody in coating buffer (cat.-no. 23-002, Chimera Biotec, Dortmund, Germany). An overview of target antigens, capture antibody and detection conjugates (Antibody-DNA conjugates) as well as the working concentrations of the capture antibodies is given in Table 1. Immobilization was carried out overnight at 4°C, the modules were subsequently washed three times with 240 µl /well buffer A (cat.-no. 23-004, Chimera, Dortmund, Germany) and blocked overnight with 240 µl / well of a blocking solution containing 150 mM NaCl, 20 mM Tris, 4,5 % Skim milk powder (Oxoid), 1 mg/ml DNA MB-grade (Roche), 5 mM EDTA (Sigma). On the following day, the modules were washed four times with buffer B (cat.-no. 23-005, Chimera) and incubated with a positive control ("PC" = 100 pg/ml) of the target antigen spiked in biological matrix (see Table 2) and a negative control ("NC") of the matrix without antigen, respectively. Positive and negative controls were either incubated as a pure, non-diluted sample ("0") or diluted in a 1:2 (10 µl sample + 20 µl Sample Dilution Buffer) ratio in various sample dilution buffers ("1" - "7" as listed in Table 3). Complete assay volume (either non-diluted or diluted sample) was uniformly 30 µl. Incubation was carried out for 60 min at room temperature and orbital shaking. Following another washing step with buffer B, all wells were incubated for 30 min with their respective antibody-DNA detection conjugates (see Table 1) in a 1:300 dilution in TBS (20 mM Tris/HCl pH 7,4, 5 mM EDTA, 0,09% Tween 20,), 0,5 % Skim milk powder (LP0031, Oxoid), 0,1 mg/ml DNA (MB grade, cat.-no. 114671490001, Roche, Mannheim). Subsequent to a final washing step (seven times buffer B, two times buffer A), a PCR amplification mix (Chimera Biotec) including a fluorescence-generating probe was added to each well, the modules were sealed an a real time PCR was carried out in a real-time PCR device (Stratagene MX3005 P) according to the program as listed in Table 4.

The instrument records the time "Ct", at which the measured fluorescence signal triggered by the amplified DNA crosses a given threshold. To calculate the signal-to-background ratio Ct_{P/N}, the difference between the absolute signal of the positive control (Ct_{P/N}) and the negative control (Ct_{NC}) was determined by subtraction of the signals: Ct_{P/N} = (Ct_{NC}-Ct_{PC}). Results for Ct_{P/N} for different buffers / target / matrix combinations are summarized in Table 5.

These results show that the separate addition of DNA or protein both improve the signal-to-background ratio Ct_{P/N}. Typically, the influence of the pure DNA is lower than the influence of the pure protein. However, the addition of a mixture of DNA and protein significantly improves the Ct_{P/N} values; this effect is different form a pure combination of both effects for DNA and protein alone. By a variation of protein and DNA concentration for buffer optimization, the effect could be further increased: Once again, the effect of a combination of DNA and protein revealed maximum efficiency.

Thereby the main effect of the CDB buffer is a decrease in assay background Ct_{NC}: In Figure 2, a comparison of the absolute signals for Ct_{PC} und CT_{NC} is shown at the example of the detection of rabbit-IgG spiked in serum and diluted in several buffers. The best signal-to-background ratio of Ct_{P/N} = 9.9 was obtained for SDB 7, containing high amounts of protein and DNA.

**Table 1: Used antibodies and antigens**

| **Antigen** | **Capture-Antibody** | **Working concentration** **of the capture antibody** | **Imperacer^{™}-Detection** **conjugate** **(Chimera Biotec)** |
|---|---|---|---|
| Rabbit-IgG (I5006, Sigma) | Goat-anti-rabbit-IgG (R4880, Sigma) | 10 µg/ml | CHI-Rabbit, cat.-no. 12-005 |
| PSA (P3235, Sigma) | Clone 8301 (Diagnostic Systems Laboratories) | 2 µg/ml | CHI-PSA cat.-no. 11-007 |
| Human Interleukin 6 (DY 206, R&D Systems) | Mouse-anti-hIL6 (DY 206, R&D Systems) | 2 µg/ml | CHI-IL6 cat.-no. 12-014 |
| Rec. Humanes PrP (Prionics) | 3F4 (Gentaur) | 5 µg/ml | CHI-PrP cat.-no. 12-125 |

**Table 2: Biological matrices**

| | **Matrix** | **Source** | **Comment** |
|---|---|---|---|
| **A** | BISEKO | Biotest | Standardized human serum |
| **B** | Plasma | Blutbank | Pooled human serum |
| **C** | Serum | Individual sample | Human serum |
| **D** | Tissue homogenate | Individual sample | Cow |
| **E** | Cell cultur media | Chimera | Own preparation |

**Table 3: Sample Dilution Buffer composition ("SDB")**

| **"SDB"-Buffer** | **Contents** |
|---|---|
| 1 | TBS / pH 7,4 / 20 mM Tris/HCl / 5 mM EDTA / 0,09% Tween 20 |
| 2 | TBS / pH 7,4 / 20 mM Tris/HCl / 5 mM EDTA / 0,09% Tween 20 0,1 mg/ml DNA (MB grade, cat.-no. 114671490001, Roche, Mannheim) |
| 3 | TBS / pH 7,4 / 20 mM Tris/HCl / 5 mM EDTA / 0,09% Tween 20 0,5 % Skim milk powder (LP0031, Oxoid) |
| 4 | TBS / pH 7,4 / 20 mM Tris/HCl / 5 mM EDTA / 0,09% Tween 20 0,5 % Skim milk powder (LP0031, Oxoid) 0,1 mg/ml DNA (MB grade, cat.-no. 114671490001, Roche, Mannheim) |
| 5 | TBS / pH 7,4 / 20 mM Tris/HCl / 5 mM EDTA / 0,09% Tween 20 0,5 mg/ml DNA (MB grade, cat.-no. 114671490001, Roche, Mannheim) |
| 6 | TBS / pH 7,4 / 20 mM Tris/HCl / 5 mM EDTA / 0,09% Tween 20 2,5 % Skim milk powder (LP0031, Oxoid) |
| 7 | TBS / pH 7,4 / 20 mM Tris/HCl / 5 mM EDTA / 0,09% Tween 20 2,5 % Skim milk powder (LP0031, Oxoid) 0,5 mg/ml DNA (MB grade, cat.-no. 114671490001, Roche, Mannheim) |

**Table 4: PCR**

| **Time** | **Temperature** | **Repeats** |
|---|---|---|
| 5 min | 95°C | 1x |
| 30 sec | 50 °C | 40x |
| 30 sec | 72°C | |
| 12 sec | 95°C | |

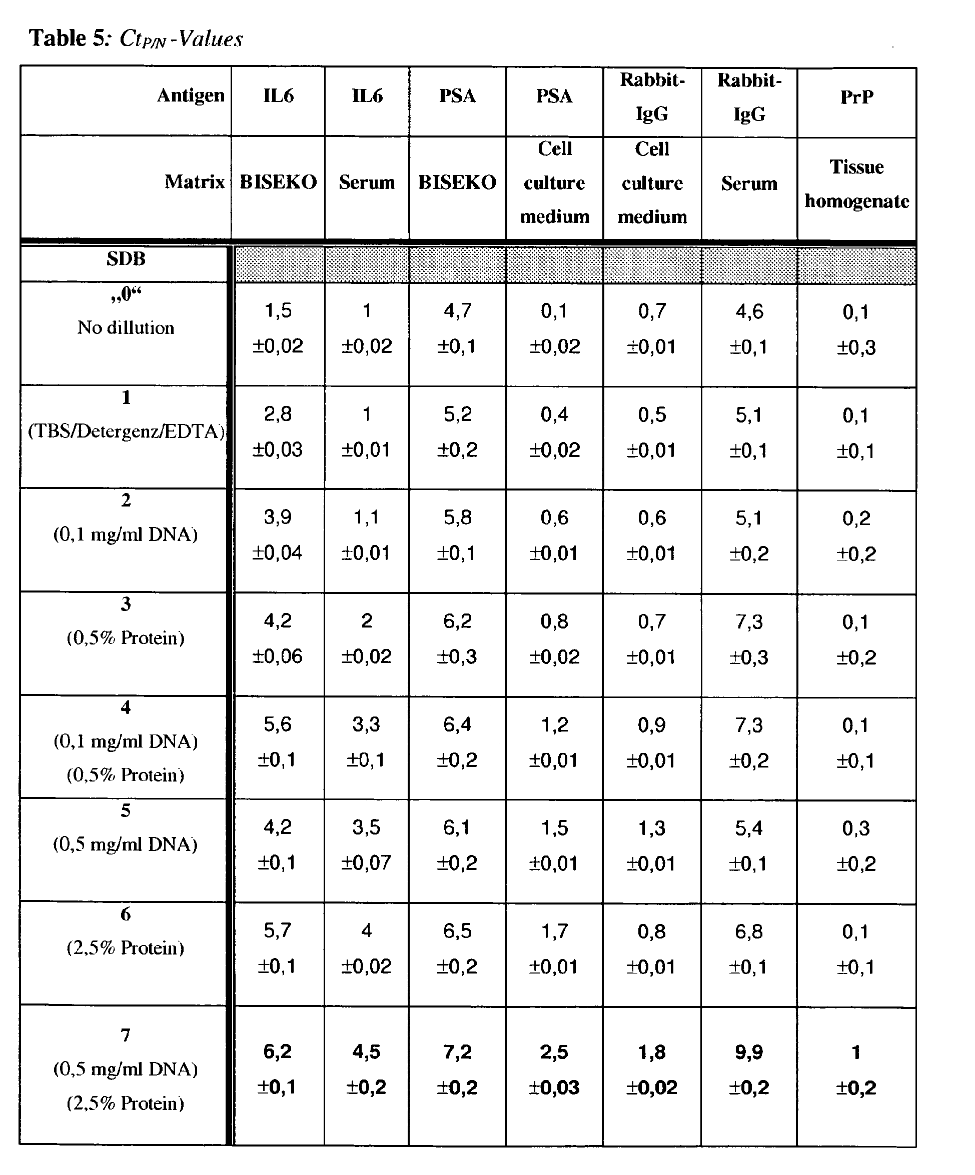

### Example 2: Direct detection of an antigen from a matrix containing compounds which inhibit binding by dilution in a sample dilution buffer

In this experiment, the target antigen rabbit-IgG was spiked as a positive control (5 ng/ml) in a cell lysate buffer containing 1.5 mg/ml plant cell homogenate protein in TBS buffer, 1% SDS and 1mM DTT. The spiked buffer was either incubated directly in TopYield modules or diluted in various dilution ratios in a sample dilution buffer ("SDB 7") containing TBS / pH 7,4 / 20 mM Tris/HCl / 5 mM EDTA / 0,09% Tween 20, 2,5 % Skim milk powder (LP0031, Oxoid), 0,5 mg/ml DNA (MB grade, cat.-no. 114671490001, Roche, Mannheim) previous to incubation. Immobilization of the target antigen solutions was carried out overnight at 4°C with an assay volume of 30 µl / well, additionally, a sample of the cell lysate buffer without target antigen was incubated in similar dilutions as a negative control. Subsequently, the modules were blocked, incubated with detection conjugate (CHI-Rabbit and detected by real time PCR as described in Example 1.

The results for positive and negative control as obtained for different dilution ratios are summarized in Table 6. It is obvious from the results that a direct IPCR without further dilution is not functional. The best Ct_{P/N} was obtained for a dilution of 1:16, indicating an optimum dilution range typical for this application.

**Table 6: Different dilutions in SDB of a matrix sample containing SDS and DTT in direct IPCR**

| | **Pure** | **1:4 in SDB 7** | **1:8 in SDB 7** | **1:16 in SDB7** | **1:32 in SDB 7** |
|---|---|---|---|---|---|
| **Ct_{NC}** | 27 | 26.5 | 27.5 | 29.5 | 29.7 |
| **Ct_{PC}** | 27 | 26.5 | 27 | 26.8 | 28 |
| **Ct_{PC}** | 0 | 0 | 0.5 | 2.7 | 1.7 |

### Example 3: Highly sensitive sandwich detection of an antigen from a biological matrix by utilization of the DNA-containing sample dilution buffer

Te evaluate the absolute sensitivity of the improved IPCR assay using sample dilution buffer ("SDB"), a dilution series of IL6 spiked in human serum was prepared and quantified according to the assay protocol as described in example 1, including a 1+1 dilution of the spiked samples in SDB 7. In parallel, a conventional ELISA was carried out, using a biotinylated anti-IL6 detection antibody (R&D Systems) in a working concentration of 200 ng/ml according to manufacturer's instruction. Following a standard washing step, the biotinylated antibody was coupled with a streptavidin / alkaline-phosphatase conjugate, diluted 1:5000 in TBS. Following a final washing step, detection was carried out using the sensitive fluorescence substrate AttopHos (Roche) and a multilabel counter.

Results are shown in Table 7: As obvious from the date, ELISA detection limit was found at 100 pg/ml while IPCR using SDB 7 dilution revealed a detection limit of 100 fg/ml. Remarkable is the extremely high intra-assay precision of the IPCR method (Standard deviation < 1% average signal), indicating the high robustness of the IPCR assay when using SDB.

**Table 7: SDB dilution series in ELISA and IPCR assay experiments**

| **Antigen concentration** **[IL6 spiked in human serum]** | **ELISA signal** **[Fluorescence a.u.]** | **IPCR signal** **[Ct]** |
|---|---|---|
| **100ng/ml** | *724125* ± *20904* | *1796* ± *0.02* |
| **10ng/ml** | *346236* ± *39951* | *19.185* ± *0,005* |
| **Ing/ml** | *52730* ± *899* | *22.28* ± *0.1* |
| **100pg/** | **8295 ± 463** | *25.57* ± *0.05* |
| **10pg/ml** | 3589 ± 221 | *27.81* ± *0.04* |
| **1pg/ml** | 3097 ± 16 | *29.235* ± *0.12* |
| **100fg/ml** | 3204 ± 178 | **29.585 ± 0.14** |
| **NC** | 3158 ± 235 | 30.215 ± 0.04 |

## Claims

1. Method for determining the presence or amount of an analyte in a sample by an Immuno-PCR, said method comprising diluting the sample with a sample dilution buffer, wherein the sample dilution buffer comprises one or more nucleic acid molecules, wherein the concentration of the nucleic acid molecules in the sample dilution buffer is between about 0.01 mg/ml and about 10 mg/ml.

2. The method of claim 1, wherein the sample is contacted with an analyte-specific binding molecule in the presence of the sample dilution buffer to form an analyte-binding molecule complex.

3. The method of claim 2, wherein the analyte-specific binding molecule is a capture molecule or a detection molecule.

4. The method of any one of claims 2-3, wherein the analyte-specific binding molecule is an antibody or antibody fragment.

5. The method of claim 1, wherein the analyte is immobilized on a solid support in the presence of the sample dilution buffer.

6. The method of any one of claims 1-5, wherein the sample is selected from the group consisting of bodily fluids, culture media, tissue samples, and cell lysates.

7. The method of any one of claims 1-6, wherein the one or more nucleic acid molecules are selected from the group consisting of DNA, RNA and PNA; and/or the one or more nucleic acid molecules are fragmented genomic DNA.

8. The method of any one of claims 1-7, wherein the sample is diluted with the buffer solution from about 0.1-fold to about 100-fold.

9. The method of any one of claims 1-8, wherein the buffer solution comprises one or more proteins and/or peptides.

10. The method of claim 9, wherein the one or more proteins and/or peptides are selected from albumins, caseins and globulins.

11. The method of any one of claims 1-10, wherein the buffer solution additionally comprises one or more compounds selected from the group of detergents, salts, buffer substances and chelating agents.

12. The method of any one of claims 1-11, wherein in the Immuno-PCR assay the buffers utilized for blocking the solid support, washing and dilution or storage of the binding molecules also contain one or more nucleic acid molecules.

13. Use of a buffer solution comprising one or more nucleic acid molecules for the dilution of an Immuno-PCR sample to reduce the background in an Immuno-PCR reaction, wherein the concentration of the nucleic acid molecules in the buffer solution is between about 0.01 mg/ml and about 10 mg/ml.

14. The use of claim 13, wherein the buffer solution comprises one or more proteins and/or peptides.

15. The use of claim 13 or 14, wherein the buffer solution additionally comprises one or more compounds selected from the group consisting of detergents, salts, buffer substances and chelating agents.

## Patentansprüche

1. Verfahren zur Bestimmung des Vorhandenseins oder der Menge eines Analyten in einer Probe durch eine Immuno-PCR, wobei das Verfahren das Verdünnen der Probe mit einem Probenverdünnungspuffer umfasst, wobei der Probenverdünnungspuffer ein oder mehrere Nukleinsäuremoleküle umfasst, wobei die Konzentration der Nukleinsäuremoleküle in dem Probenverdünnungspuffer zwischen ungefähr 0,01 mg/ml und ungefähr 10 mg/ml beträgt.

2. Das Verfahren nach Anspruch 1, wobei die Probe mit einem Analyt-spezifischen Bindungsmolekül in Gegenwart des Probenverdünnungspuffers in Kontakt gebracht wird, um einen Analyt-Bindungsmolekülkomplex zu bilden.

3. Das Verfahren nach Anspruch 2, wobei das Analyt-spezifische Bindungsmolekül ein Einfangmolekül oder ein Nachweismolekül ist.

4. Das Verfahren nach einem der Ansprüche 2-3, wobei das Analyt-spezifische Bindungsmolekül ein Antikörper oder ein Antikörperfragment ist.

5. Das Verfahren nach Anspruch 1, wobei der Analyt in Gegenwart des Probenverdünnungspuffers auf einem festen Träger immobilisiert wird.

6. Das Verfahren nach einem der Ansprüche 1-5, wobei die Probe ausgewählt wird aus der Gruppe bestehend aus Körperflüssigkeiten, Kulturmedien, Gewebeproben und Zelllysaten.

7. Das Verfahren nach einem der Ansprüche 1-6, wobei das eine oder die mehreren Nukleinsäuremoleküle ausgewählt wird/werden aus der Gruppe bestehend aus DNA, RNA und PNA; und/oder das eine oder die mehreren Nukleinsäuremoleküle fragmentierte genomische DNA ist/sind.

8. Das Verfahren nach einem der Ansprüche 1-7, wobei die Probe mit der Pufferlösung von ungefähr 0,1-fach bis ungefähr 100-fach verdünnt wird.

9. Das Verfahren nach einem der Ansprüche 1-8, wobei die Pufferlösung ein oder mehrere Proteine und/oder Peptide umfasst.

10. Das Verfahren nach Anspruch 9, wobei das eine oder die mehreren Proteine und/oder Peptide ausgewählt werden aus Albuminen, Caseinen und Globulinen.

11. Verfahren nach einem der Ansprüche 1-10, wobei die Pufferlösung zusätzlich eine oder mehrere Verbindungen umfasst, die aus der Gruppe von Detergenzien, Salzen, Puffersubstanzen und Chelatisierungsmitteln ausgewählt werden.

12. Das Verfahren nach einem der Ansprüche 1-11, wobei in dem Immuno-PCR Assay die Puffer, die für das Blockieren des festen Trägers, das Waschen und die Verdünnung oder Aufbewahrung der Bindungsmoleküle verwendet werden, ebenfalls ein oder mehrere Nukleinsäuremoleküle enthalten.

13. Verwendung einer Pufferlösung, die ein oder mehrere Nukleinsäuremoleküle umfasst, für die Verdünnung einer Immuno-PCR Probe, um den Hintergrund in einer Immuno-PCR Reaktion zu verringern, wobei die Konzentration der Nukleinsäuremoleküle in der Pufferlösung zwischen ungefähr 0,01 mg/ml und ungefähr 10 mg/ml beträgt.

14. Die Verwendung nach Anspruch 13, wobei die Pufferlösung ein oder mehrere Proteine und/oder Peptide umfasst.

15. Die Verwendung nach Anspruch 13 oder 14, wobei die Pufferlösung zusätzlich eine oder mehrere Verbindungen umfasst, die aus der Gruppe bestehend aus Detergenzien, Salzen, Puffersubstanzen und Chelatisierungsmitteln ausgewählt werden.

## Revendications

1. Procédé pour déterminer la présence ou la quantité d'un analyte dans un échantillon par une immuno-PCR, ledit procédé comprenant la dilution de l'échantillon avec un tampon de dilution d'échantillon, où le tampon de dilution d'échantillon comprend une ou plusieurs molécules d'acide nucléique, où la concentration des molécules d'acide nucléique dans le tampon de dilution d'échantillon est comprise entre environ 0,01 mg/ml et environ 10 mg/ml.

2. Procédé selon la revendication 1, où l'échantillon est mis en contact avec une molécule de liaison spécifique à un analyte en présence du tampon de dilution d'échantillon pour former un complexe analyte-molécule de liaison.

3. Procédé selon la revendication 2, où la molécule de liaison spécifique à un analyte est une molécule de capture ou une molécule de détection.

4. Procédé selon l'une quelconque des revendications 2 à 3, où la molécule de liaison spécifique à un analyte est un anticorps ou un fragment d'anticorps.

5. Procédé selon la revendication 1, où l'analyte est immobilisé sur un support solide en présence du tampon de dilution d'échantillon.

6. Procédé selon l'une quelconque des revendications 1 à 5, où l'échantillon est choisi parmi le groupe consistant en fluides corporels, milieux de culture, échantillons de tissus, et lysats de cellules.

7. Procédé selon l'une quelconque des revendications 1 à 6, où l'une ou plusieurs molécules d'acide nucléique sont choisies parmi le groupe consistant en ADN, ARN et ANP ; et/ou l'une ou plusieurs molécules d'acide nucléique sont des ADN génomiques fragmentés.

8. Procédé selon l'une quelconque des revendications 1 à 7, où l'échantillon est dilué avec la solution tampon d'environ 0,1 fois à environ 100 fois.

9. Procédé selon l'une quelconque des revendications 1 à 8, où la solution tampon comprend une ou plusieurs protéines et/ou peptides.

10. Procédé selon la revendication 9, où l'une ou plusieurs protéines et/ou peptides sont choisis parmi des albumines, caséines et globulines.

11. Procédé selon l'une quelconque des revendications 1 à 10, où la solution tampon comprend de manière supplémentaire un ou plusieurs composés choisis parmi le groupe consistant en détergents, sels, substances tampon et agents chélatants.

12. Procédé selon l'une quelconque des revendications 1 à 11, où dans l'essai d'immuno-PCR les tampons utilisés pour le blocage du support solide, le lavage et la dilution ou le stockage des molécules de liaison contiennent également une ou plusieurs molécules d'acide nucléique.

13. Utilisation d'une solution tampon comprenant une ou plusieurs molécules d'acide nucléique pour la dilution d'un échantillon d'immuno-PCR pour réduire le bruit de fond dans une réaction d'immuno-PCR, où la concentration des molécules d'acide nucléique dans la solution tampon est comprise entre environ 0,01 mg/ml et environ 10 mg/ml.

14. Utilisation selon la revendication 13, où la solution tampon comprend une ou plusieurs protéines et/ou peptides.

15. Utilisation selon la revendication 13 ou 14, où la solution tampon comprend de manière supplémentaire un ou plusieurs composés choisis parmi le groupe consistant en détergents, sels, substances tampon et agents chélatants.
